# EUROPÄISCHE PATENTANMELDUNG

(11) **EP 2 023 258 A1**
(43) Veröffentlichungstag der Anmeldung: **11.02.2009**
(21) Anmeldenummer: 08159445.9
(22) Anmeldetag: 01.07.2008
(51) Int. Cl.: G06F 19/00

(54) **Anordnung und Verfahren für die Fernprogrammierung eines programmierbaren persönlichen Gerätes**

(30) Priorität: 19.07.2007 DE 102007033993
(71) Anmelder: BIOTRONIK CRM Patent AG, 6341 Baar (CH)
(72) Erfinder: Hennig, Carsten, 13591 Berlin (DE)
(74) Vertreter: Lindner-Vogt, Karin L.

(57) **Zusammenfassung**

Die Erfindung betrifft eine Anordnung und ein Verfahren für die Fernprogrammierung eines programmierbaren persönlichen Gerätes (10, 10'), insbesondere eines implantierbaren medizinischen Gerätes wie einem Herzschrittmacher, Defibrillator oder dergleichen, wobei ein Auswählen (61) eines persönlichen Gerätes, ein Zusammenstellen (63) eines Programmierauftrags (50) durch Entgegennehmen von Eingaben eines Benutzers und Überprüfen (63) der Eingaben auf Plausibilität und Kompatibilität mit dem persönlichen Gerät in einem Programmiergerät (20), ein Übertragen (65) des Programmierauftrags an ein auf das persönliche Gerät abgestimmtes Patientengerät (30) über eine wenigstens mittelbare Verbindung, ein Empfangen (67) des Programmierauftrags durch das Patientengerät und Übertragen des Programmierauftrags an das persönliche Gerät, und ein Entgegennehmen (69) des Programmierauftrags durch eine programmierbare Steuerung des persönlichen Gerätes vorgesehen sind.

## Beschreibung

Die Erfindung betrifft eine Anordnung für die Fernprogrammierung eines programmierbaren persönlichen Gerätes, insbesondere eines implantierbaren medizinischen Gerätes wie einen Herzschrittmacher, Defibrillator oder dergleichen.

Um eine möglichst optimale Versorgung des Patienten und einen optimalen Betrieb beispielsweise eines Herzschrittmachers sicherzustellen, ist es in der Praxis oft notwendig, auf Basis des Nachsorgeverlaufes und der aktuellen Konstitution des Patienten die Programmierung eines Herzschrittmachers oder eines ähnlichen Gerätes anzupassen.

Bei bekannten Lösungen wird zur Umprogrammierung des Implantats ein Programmiergerät verwendet, das ein geändertes Programm für das Implantat über eine zumeist schnurlose Datenkommunikation direkt in das Implantat überträgt. Dazu müssen Programmiergerät und Implantat räumlich nah beieinander sein. Dies macht es nötig, dass der Patient seinen Arzt, ein Krankenhaus oder eine andere Nachsorgestation aufsucht, also einen Ort, an dem das Programmiergerät vorgehalten wird. Damit ergibt sich die Notwendigkeit einer zeitlichen und räumlichen Synchronisierung von Arzt und Patient.

Diese Notwendigkeit wird oft als lästig empfunden und schränkt insbesondere die Mobilität des Patienten nicht unwesentlich ein.

Aufgabe der vorliegenden Erfindung ist es, eine Anordnung für die Fernprogrammierung eines programmierbaren persönlichen Gerätes bereitzustellen, bei dem eine Programmübertragung in das programmierbare persönliche Gerät möglich ist, ohne dass Arzt und Patient zusammenkommen müssen. Insbesondere soll dem Arzt hierbei die Möglichkeit gegeben bleiben, das für ihn vertraute Programmiergerät zu verwenden.

Erfindungsgemäß wird diese Aufgabe gelöst durch eine Anordnung für die Fernprogrammierung, wie sie im Anspruch 1 definiert ist, sowie ein Verfahren für die Fernprogrammierung, wie es in Anspruch 19 definiert ist. Ein erfindungsgemäßes Programmiergerät, ein erfindungsgemäßes Patientengerät und ein erfindungsgemäßes Servicecenter ist jeweils in einem der Ansprüche 16 bis 18 definiert.

Die Erfindung basiert auf der Einsicht, dass ein Patientengerät, wie es herkömmlich bereits zur Übermittlung von Telemetriedaten eines Gerätes wie einem Herzschrittmacher an ein Servicecenter verwendet wird, mit einer weiteren Funktionalität einer Relaisstation ausgerüstet werden kann, um einen in einem Programmiergerät erstellten Programmauftrag in das persönliche Gerät zu übermitteln, ohne dass ein Aufsuchen des Programmiergerätes durch den Träger des persönlichen Gerätes nötig wäre. Das Patientengerät befindet sich entfernt von dem Programmiergerät in einem Bereich, der zumindest regelmäßig vom Träger des persönlichen Gerätes aufgesucht wird, sodass das Patientengerät in effektiver Weise als Zwischenstation für die Fernprogrammierung des persönlichen Gerätes verwendet werden kann.

Bevorzugte Ausführungsformen der Erfindung sind in den untergeordneten Ansprüchen definiert.

Weitere vorteilhafte Ausgestaltungen der Erfindung ergeben sich durch Kombination von Merkmalen der Ansprüche sowie aus der nachfolgenden Beschreibung bevorzugter Ausführungsbeispiele.

Die Erfindung wird im Folgenden anhand bevorzugter Ausführungsbeispiele mit Bezug auf die beiliegenden Figuren näher erläutert. Hierbei zeigt:
- Fig. 1: eine erste erfindungsgemäße Anordnung zur Fernprogrammierung eines Implantats,
- Fig. 2: eine zweite Ausführung einer erfindungsgemäßen Anordnung zur Fernprogrammierung eines Implantats,
- Fig. 3: eine dritte Ausführung einer erfindungsgemäßen Anordnung für die Fernprogrammierung eines Implantats,
- Fig. 4: einen in der in Fig. 3 gezeigten Anordnung verwendeten Datensatz,
- Fig. 5: einen schematischen Ablauf eines erfindungsgemäßen Verfahrens für die Fernprogrammierung eines programmierbaren persönlichen Gerätes,
- Fig. 6: einen schematischen Ablauf einer erfindungsgemäßen Fernprogrammierung,
- Fig. 7: einen weiteren Ablauf einer erfindungsgemäßen Fernprogrammierung und
- Fig. 8: einen dritten Ablauf einer erfindungsgemäßen Fernprogrammierung.

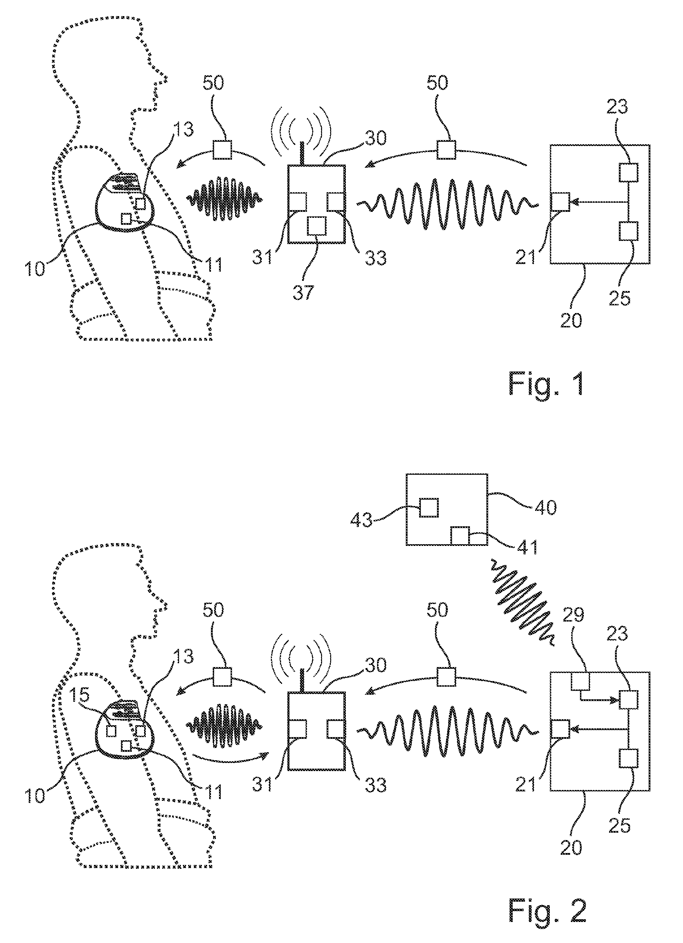

Fig. 1 zeigt schematisch einen Patienten mit einem Implantat 10. In der Nähe des Patienten und damit in der Nähe des Implantats 10 ist ein Patientengerät 30 angeordnet. Entfernt vom Patienten und vom Patientengerät 30 befindet sich das Programmiergerät 20. Programmiergerät 20 und Patientengerät 30 sind zumindest für die Dauer der Fernprogrammierung auf das jeweilige Implantat 10 abgestimmt.

Das Implantat 10 weist eine erste Schnittstelle 11 und eine programmierbare Steuerung 13 auf. Vorzugsweise weist das Implantat 10 zusätzlich eine Authentifizierungseinheit auf.

Das Patientengerät 30 ist mit einer ersten Schnittstelle 31 und einer zweiten Schnittstelle 33 ausgestattet. Vorzugsweise weist das Patientengerät 30 zusätzlich eine Authentifizierungseinheit 37 auf.

Das Programmiergerät 20 besitzt eine erste Schnittstelle 21, eine Programmiereinheit 23 und eine Eingabeeinheit 25.

Die Eingabeeinheit 25 ist dazu ausgelegt, Eingaben eines Benutzers entgegenzunehmen, wobei die Eingabeeinheit 25 mit der Programmiereinheit 23 gekoppelt ist, um mit dieser zusammen einen Programmierauftrag zusammenzustellen. Die Programmiereinheit 23 ist ausgebildet, auf Basis der Eingaben ein neues oder ein geändertes Programm mit einem geeigneten Satz von Steuerparametern für das persönliche Gerät zu erzeugen und dieses Programm auf Plausibilität und Kompatibilität mit dem persönlichen Gerät hin zu überprüfen. Nur ein Programm, das den jeweilig gewünschten Anforderungen an Plausibilität und Kompatibilität gerecht wird, wird für einen Programmierauftrag zugelassen.

Die erste Schnittstelle 21 des Programmiergerätes und die zweite Schnittstelle des Patientengerätes sind vorliegend für eine Übertragung eines Datensatzes 50 vom Programmiergerät 20 zum Patientengerät 30 ausgestaltet. Gemäß Fig. 1 sind die Schnittstellen 21, 33 für eine Verbindung gemäß einer GSM-Spezifikation, also einer drahtlosen Funkübertragung, ausgestaltet. Alternativ dazu kann auch eine Verbindung über eine landgestützte Telefonleitung oder eine andere geeignete Kommunikationsart eingesetzt werden. Hierbei sind auch Kombinationen aus unterschiedlichen Protokollen beziehungsweise Spezifikationen möglich. Beispielsweise kann die Kommunikation auch über ein WLAN-Netz, an das das Programmiergerät angeschlossen ist, eine landgestützte Datenleitung und eine Bluetooth-Verbindung zwischen der landgestützten Datenleitung und dem Patientengerät 30 erfolgen, wobei unidirektionale oder bidirektionale Verbindungen möglich sind.

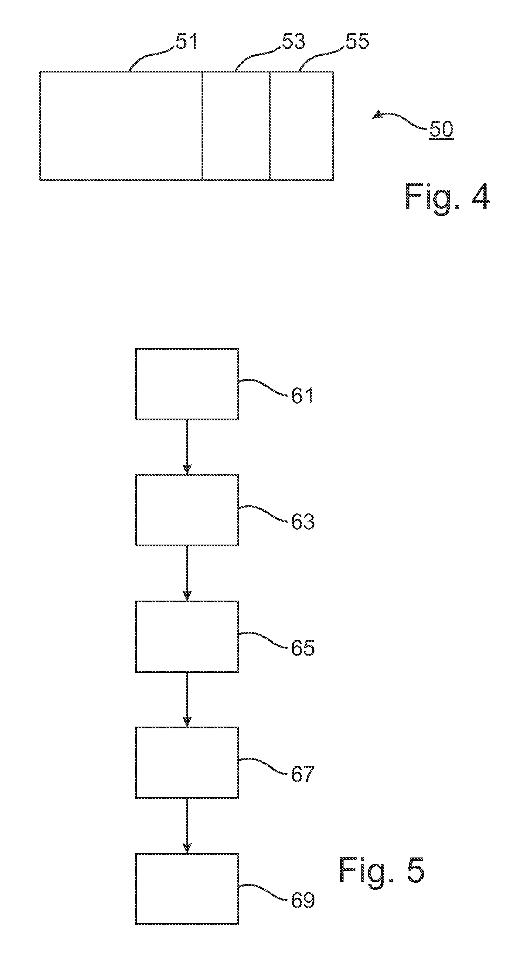

Das Patientengerät 30 empfängt den Datensatz 50, wobei die Authentifizierungseinheit 37 den Datensatz 50 auf Vorliegen einer gültigen Authentifizierung überprüft. Nur wenn das Vorliegen einer gültigen Authentifizierung im Datensatz 50 durch die Authentifizierungseinheit 37 bestätigt ist, wird der Datensatz 50 mit dem Programmierauftrag (siehe Fig. 4) über die erste Schnittstelle 31 des Patientengeräts 30 und die erste Schnittstelle 11 des Implantats 10 an das Implantat 10 übermittelt. Hierbei kann vorgesehen sein, dass lediglich der Teil des Datensatzes 50 übermittelt wird, der dem Programmierauftrag entspricht, das Authentifizierungselement des Datensatzes 50 also nicht mit übermittelt wird. Der Programmierauftrag wird von der programmierbaren Steuerung 13 des Implantats 10 empfangen und in geeigneter und bekannter Weise verarbeitet.

Fig. 2 zeigt ein weiteres Ausführungsbeispiel einer erfindungsgemäßen Anordnung für die Fernprogrammierung. In Teilen entspricht die in Fig. 2 gezeigte Anordnung der in Fig. 1 gezeigten Anordnung, sodass auf eine Beschreibung einander entsprechender Elemente verzichtet werden kann. In einem Aspekt unterscheidet sich die in Fig. 2 gezeigt Ausführungsform von der Anordnung aus Fig. 1: Die Anordnung weist ein Servicecenter 40 mit einer ersten Schnittstelle 41 und einem Datenspeicher 43 auf. Die Schnittstelle 41 dient zum Verbinden des Servicecenters 40 mit dem Programmiergerät 20. Der Datenspeicher 43 enthält Daten, die sich auf das Implantat 10 beziehen.

Das Programmiergerät 20 weist eine zweite Schnittstelle 29 auf, die zum Herstellen einer Verbindung mit dem Servicecenter 40 vorgesehen ist.

Bei der Erstellung und Prüfung eines Programmierauftrages wird über die Schnittstellen 29 und 41 eine Abfrage an das Servicecenter gerichtet, in dem Datenspeicher 43 gespeicherte Daten bezüglich des Implantats 10 an das Programmiergerät 20 zu übermitteln.

Unter Verwendung der aus dem Datenspeicher 43 übermittelten Daten, wird in an sich bekannter Weise, der Programmierauftrag erstellt.

Die Übermittlung des Programmierauftrages, der in den Datensatz 50 eingebettet ist, erfolgt entsprechend zu der Übermittlung, wie sie mit Bezug auf Fig. 1 beschrieben wurde.

Das Implantat 10 weist in der hier vorliegenden Ausführungsform eine Abgleichseinheit 15 auf. Bei der Erstellung des Programmierauftrages und der Beschaffung der Daten aus dem Datenspeicher 43 wird eine Identifikation eines vorherigen Programms des Implantats 10 erzeugt, die dem Programmauftrag im Datensatz 50 beigegeben ist. Die Abgleichseinheit 15 ist ausgebildet, diese dem Programmierauftrag beigegebene Identifikationen mit dem aktuellen Programm der Steuerung abzugleichen. Sollte dieser Abgleich ergeben, dass der Programmauftrag nicht auf Basis des aktuellen, im Implantat aktiven Programms erstellt wurde, wird der Programmierauftrag zurückgewiesen. Ergibt andererseits der Abgleich, dass das aktuelle Programm, oder ein dem aktuellen Programm entsprechendes vorheriges Programm, Grundlage für den Programmierauftrag war, so wird der Programmierauftrag angenommen und von der Steuerung 13 abgearbeitet. Eine Information zu dem Abgleichsergebnis beziehungsweise zu der Annahme oder Abweisung des Programmierauftrages wird über die Schnittstellen 11 und 31 an das Patientengerät 30 übermittelt. Hier kann diese Information beispielsweise für eine spätere Abfrage gespeichert oder direkt an eine geeignete Stelle zur Kontrolle übermittelt werden.

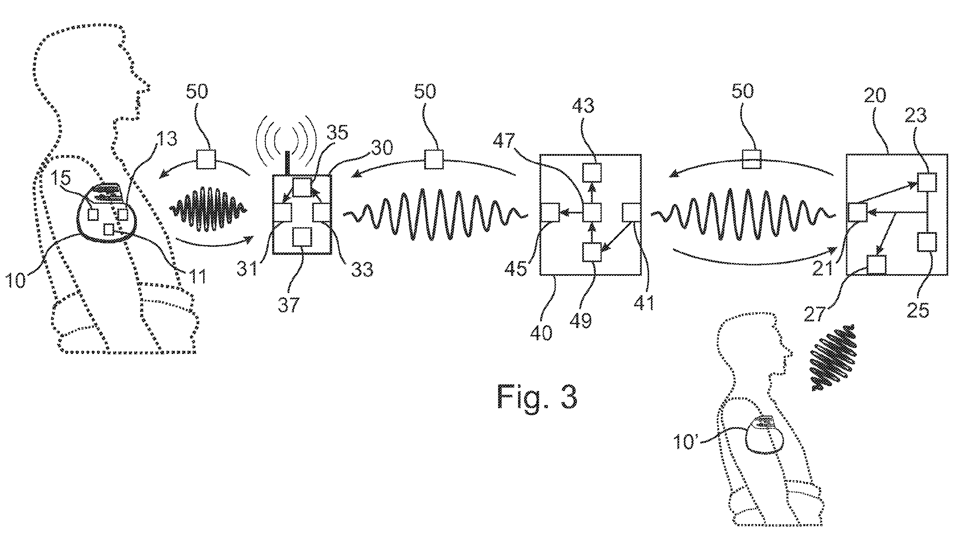

Fig. 3 zeigt eine weitere Ausführungsform der vorliegenden Erfindung.

Der Patient ist mit einem Implantat 10 ausgestattet, das eine erste Schnittstelle 11, eine Steuerung 13 und eine Abgleichseinheit 15 aufweist. Das Implantat 10 entspricht dem in Fig. 2 gezeigten Implantat.

Das Patientengerät 30 weist eine erste Schnittstelle 31, eine zweite Schnittstelle 33, einen Programmspeicher 35 und eine Authentifizierungseinheit 37 auf. Erste und zweite Schnittstelle 31, 33 und Authentifizierungseinheit 37 entsprechen den zuvor beschriebenen Einheiten. Der Programmspeicher 35 ist mit der Schnittstelle 33 verbunden und dient dazu, einen empfangenen Datensatz beziehungsweise einen empfangenen Programmierauftrag zumindest temporär zwischenzuspeichern. Der zwischengespeicherte Programmierauftrag beziehungsweise der zwischengespeicherte Datensatz wird zu einem geeigneten Zeitpunkt über die Schnittstelle 31 an das Implantat 10 weitergeleitet. Auf diese Weise ist es dem Patientengerät möglich, einen Programmierauftrag zu empfangen und diesen solange vorzuhalten, bis der Patient mit dem Implantat 10 wieder in den Kommunikationsbereich der Schnittstelle 31 kommt, sodass auch bei einer Abwesenheit des Patienten der Programmierauftrag am Patientengerät anlangen kann und bei Rückkehr des Patienten beziehungsweise des Implantats zum Patientengerät an das Implantat übermittelt wird.

Gemäß der in Fig. 3 dargestellten Ausführungsform ist das Servicecenter 40 als weitere Zwischenstation zwischen dem Programmiergerät 20 und dem Patientengerät 30 vorgesehen. Das Servicecenter 40 weist eine erste Schnittstelle 41, eine zweite Schnittstelle 45, einen Datenspeicher 43, eine Dateneinheit 47 und eine Verifikationseinheit 49 auf. Die Schnittstelle 41 dient zur Kommunikation mit dem Programmiergerät 20. Über die Schnittstelle 45 in Kombination mit der zweiten Schnittstelle des Patientengeräts 33 kann eine Verbindung zwischen dem Servicecenter 40 und dem Patientengerät 30 aufgebaut werden, um einen Datensatz 50 vom Servicecenter 40 an das Patientengerät 30 übermitteln zu können.

Über die Schnittstelle 41 empfängt das Servicecenter 40 einen Programmierauftrag, der in einem Datensatz 50 eingebettet ist. Der empfangene Datensatz beziehungsweise der empfangene Programmierauftrag wird von der Dateneinheit 47 analysiert, wobei vorbestimmte Daten auf Basis des Programmierauftrages zu dem im Datenspeicher 43 gespeicherten Daten hinzugefügt werden, die ebenso dem persönlichen Gerät zugehörig sein können. Auf diese Weise kann beispielsweise eine Dokumentation der an das Implantat 10 gesandten Programmieraufträge im Datenspeicher 43 nachgehalten werden. Die Verifikationseinheit 49 ist in der in Fig. 3 dargestellten Ausführungsform der Dateneinheit vorgeschaltet und ist dazu ausgebildet, eine Verifikation des Programmiergerätes durchzuführen, bevor die Daten des Programmierauftrages weiter verarbeitet werden. Dies erhöht die allgemeine Sicherheit, sodass absichtliche oder unabsichtliche Fehlbedienungen vermieden werden können. Der von Verifikationseinheit 49 und Dateneinheit 47 verarbeitete beziehungsweise kontrollierte Datensatz beziehungsweise Programmierauftrag ist dazu vorgesehen, über die zweite Schnittstelle 45 des Servicecenters 40 an das Patientengerät 30 übermittelt zu werden.

Das Programmiergerät 20 weist eine erste Schnittstelle 21, eine dritte Schnittstelle 27, eine Programmiereinheit 23 und eine Eingabeeinheit 25 auf. Programmiereinheit 23 und Eingabeeinheit 25 sind, ähnlich zu der mit Fig. 2 beschriebenen Ausführungsform, dazu ausgebildet, von dem Servicecenter 40 Daten mit Bezug auf das Implantat 10 anzufordern und zu empfangen. Hierbei erfolgt mittels der Verifikationseinheit 49 eine Verifikation der Datenanforderung beziehungsweise der Berechtigung des Programmiergeräts die gewünschten Daten zu erhalten. Erlaubt die Verifikationseinheit 49 die Übermittlung der Daten, werden die aus dem Datenspeicher 43 ausgelesenen Daten über die mit den Schnittstellen 41 und 21 aufgebaute Verbindung zwischen dem Servicecenter 40 und dem Programmiergerät 20 an das Programmiergerät 20 übermittelt. Die Daten werden vom Programmiergerät 20 genauer von der Programmiereinheit 23 und der Eingabeeinheit 25 zur Erstellung eines Programmierauftrages verwendet, ähnlich zu der Ausführungsform gemäß Fig. 2.

Die dritte Schnittstelle 27 des Programmiergeräts 20 ist für einen Verbindungsaufbau mit einem in der Nähe befindlichen Implantat 10' ausgestaltet und dient zur Übermittlung eines von dem Programmiergerät 20 erstellten Programmierauftrages für das Implantat 10' an das Implantat 10'. Auch für die Erstellung des Programmierauftrags für das Implantat 10' kann eine Datenabfrage im Datenspeicher 43 des Servicecenters 40 in der oben beschriebenen Weise vorgesehen sein. Das in Fig. 3 dargestellte Programmiergerät weist eine doppelte Funktionalität auf. Einerseits kann es zur Fernprogrammierung über das Servicecenter 40 und das Patientengerät 30 an das Implantat 10 eingesetzt werden. Andererseits kann ein Implantat 10' direkt durch das Programmiergerät 20 programmiert werden.

Verschiedene Ausführungsformen einer Anordnung für die Fernprogrammierung etwa eines Implantats, beispielsweise die in den Fig. 1 bis 3 dargestellten Ausführungsformen, können als alternative Ausführungen in einer gemeinsamen Anordnung für die Fernprogrammierung kombiniert sein. In dieser kombinierten Anordnung stehen dann unterschiedliche und einander ergänzende Kommunikationswege für die Übermittlung beziehungsweise Übertragung des Programmierauftrags von dem Programmiergerät an das Implantat zur Verfügung. Zudem können auch mehrere Implantate sowie mehrere Patientengeräte in einer erfindungsgemäßen Anordnung vorgesehen sein, wobei je nach Implantat beziehungsweise je nach Patientengerät unterschiedliche Kommunikationswege beziehungsweise Ausführungsformen der Anordnung Anwendung finden können. Ebenso können mehrere Servicecenter nebeneinander vorgesehen sein, die optional miteinander kommunizieren und somit ein modular aufgebautes Servicecenter bilden können.

Natürlich kann die erfindungsgemäße Anordnung auch mehrere Programmiergeräte vorsehen, die nebeneinander oder alternativ Anwendung finden.

In Fig. 4 ist schematisch ein Datensatz 50 dargestellt, der in der dargestellten Form beispielsweise in einer Anordnung, wie sie in Fig. 3 gezeigt ist, Anwendung finden kann. Der Datensatz 50 umfasst einen Programmierauftrag 51, eine Identifikation 53 und eine Authentifizierung 55. Zur Authentifizierung ist das Patientengerät 30 und / oder das persönliche Gerät 10, 10' mit einer Authentifizierungseinheit 37 ausgestattet. Die Identifikation 53 kann beispielsweise eine eindeutige Programmnummer sein, die auf ein vorheriges Programm verweist. Die Authentifizierung kann beispielsweise in der Angabe eines Passwortes oder der Kombination einer Benutzerkennung und eines Passwortes bestehen. Andere dem Fachmann geläufige Identifikationen oder Authentifizierungen sind ebenfalls möglich.

Fig. 5 zeigt schematisch einen erfindungsgemäßen Verfahrensablauf eines Verfahrens für die Fernprogrammierung eines programmierbaren persönlichen Gerätes. Nach Auswählen eines persönlichen Gerätes (Schritt 61) wird ein Programmierauftrag erstellt, in dem Benutzereingaben zusammengebracht werden. Der Programmierauftrag wird dabei zudem auf Plausibilität und Kompatibilität mit dem ausgewählten persönlichen Gerät überprüft, sodass nur ein plausibler und kompatibler Programmierauftrag weiterverarbeitet wird (Schritt 63). Der erstellte Programmierauftrag wird an ein auf das persönliche Gerät abgestimmtes Patientengerät übertragen, wobei je nach Anwendung eine mittelbare Verbindung genutzt wird, also Zwischenstationen vorhanden sind, oder eine unmittelbare Verbindung zwischen dem Patientengerät und dem Programmiergerät hergestellt wird (Schritt 65). Der Programmierauftrag wird durch das Patientengerät empfangen und an das persönliche Gerät übertragen (Schritt 67). Schließlich nimmt eine programmierbare Steuerung des persönlichen Gerätes den Programmierauftrag entgegen, was eine Bearbeitung beziehungsweise eine Abweisung des Programmierauftrages beinhaltet (Schritt 69).

In einem weiteren Ausführungsbeispiel der vorliegenden Erfindung ist das Programmiergerät per WLAN oder kabelgebundenes LAN an ein Kliniknetzwerk angebunden und hat über das Kliniknetzwerk eine Verbindung zu einem Home-Monitoring-Service-Center-Server (HMSC-Server). Der Arzt gibt am Programmiergerät seine HMSC-Zugangsdaten ein, woraufhin das Programmiergerät dem Arzt eine Liste der Patienten präsentiert, die im HMSC dem Arzt zugeordnet und die für eine Fernprogrammierung freigeschaltet sind. Nach der Auswahl des entsprechenden Patienten beziehungsweise des entsprechenden Implantats wird in der für den Arzt gewohnten Benutzeroberfläche das letzte bekannte Programm des Implantats angezeigt, wobei das Programmiergerät das letzte Programm vom HMSC übermittelt bekommen oder selbst gespeichert haben kann. Nach der Änderung beziehungsweise Anpassung des Programms wird dieses vom Programmiergerät an das HMSC übertragen und von dort an das Patientengerät des entsprechenden Patienten übertragen. Hierbei wird eine eindeutige Programmnummer der alten, d. h. noch derzeitigen Programmierung mitgeschickt. Das neue Programm auf Basis des übermittelten Programmierauftrags wird dann im Implantat nur akzeptiert, wenn die im Implantat selbst gespeicherte beziehungsweise bestimmte Programmnummer der mitgesandten Programmnummer entspricht.

Gemäß einem weiteren alternativen oder ergänzenden Aspekt ist das Programmiergerät über eine Mobilfunkverbindung, beispielsweise per GSM, direkt mit dem Server des HMSC verbunden, wobei der Ablauf der Fernprogrammierung wie oben beschrieben erfolgt. Als weitere alternative beziehungsweise Ergänzung kann sich das Programmiergerät beispielsweise über eine Mobilfunkverbindung oder das Internet direkt mit dem Patientengerät verbinden. Hierbei entfällt der Umweg über den Server des HMSC, wodurch das HMSC als Element der Zugangskontrolle und der Dokumentation der Umprogrammierung zunächst entfällt. Damit kann das HMSC zunächst keine Information über die aktuelle Programmierung liefern. Es ist jedoch möglich, dass parallel zu der Verbindung mit dem Patientengerät eine weitere Verbindung mit dem HMSC erstellt wird, die dann beispielsweise für eine Dokumentation der Umprogrammierung oder eine ergänzende Zugangskontrolle verwendet werden kann. Damit das Programmiergerät sich direkt mit dem Patientengerät verbinden kann, benötigt das Programmiergerät eine Kennnummer, beispielsweise eine Rufnummer des Patientengeräts. Vorzugsweise wurde das gewünschte Implantat bereits einmal zuvor mit dem Programmiergerät in direkter Weise programmiert und zusammen mit dem Patientengerät auf die Fernprogrammierung eingerichtet, wobei beispielsweise ein Passwort-Schutz oder eine sonstige Zugangskontrolle eingerichtet werden kann.

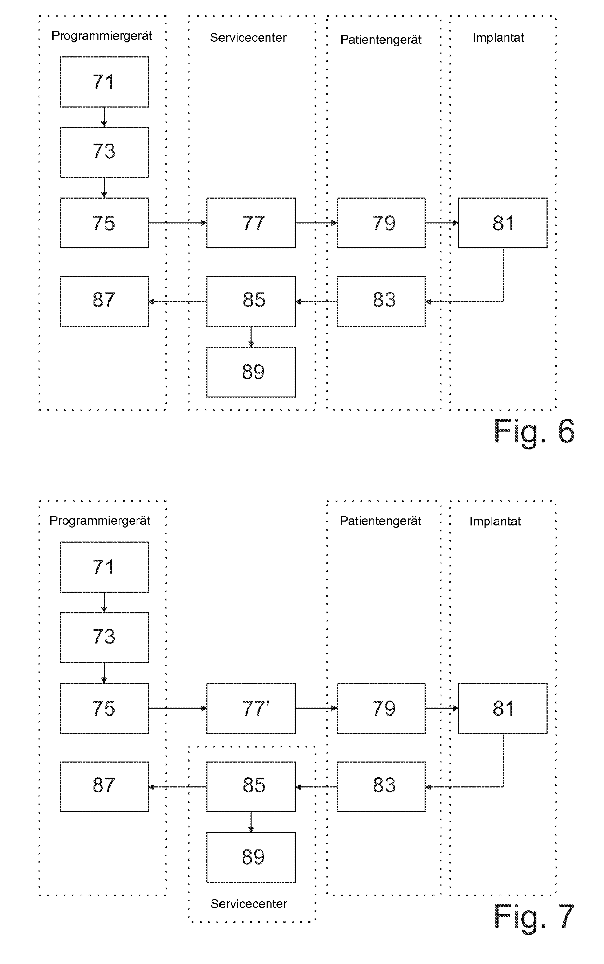

Fig. 6 zeigt schematisch einen Verfahrensablauf bei einer asynchronen Fernprogrammierung durch das Programmiergerät über ein Servicecenter. In Schritt 71 wählt ein Arzt ein zuvor abgefragtes Implantat für eine Fernprogrammierung aus. In Schritt 73 werden Parameteränderungen einschließlich einer Plausibilitätsprüfung, beispielsweise auf Parameterkonflikte, sowie eine Kompatibilitätsprüfung durchgeführt. Damit ergibt sich ein geeigneter Programmierauftrag. In Schritt 75 gibt der Arzt seine Zugangsdaten für das Servicecenter, beispielsweise ein HMSC, ein und schickt den neuen Parametersatz, also den Programmierauftrag, ab. Eine Übertragung an das Servicecenter kann beispielsweise per LAN, WLAN, GSM oder ähnlicher Kommunikationswege erfolgen. Im Servicecenter wird in Schritt 77 die Berechtigung geprüft und der Programmierauftrag zur Weiterleitung verarbeitet und in der HMSC-Datenbank dokumentiert. Per Mobilfunk oder per Festnetz-Telefonleitung, beispielsweise also per GSM oder per land-line, werden die benötigten Daten an das Patientengerät weitergeleitet. In Schritt 79 nimmt das Patientengerät das Programm an und wartet auf den nächsten Kontakt zum Zielimplantat. Mit dem nächsten Kontakt zum Zielimplantat wird der Programmauftrag vorzugsweise per MICS (Medical Implant Communications Service) an das Implantat übermittelt. In Schritt 81 wird im Implantat die Gültigkeit des Programms geprüft und das Programm gegebenenfalls aktiviert. Das Programm kann auch eine nur zeitweise oder temporäre Aktivierung vorsehen. Entsprechend des Ausgangs der Gültigkeitsprüfung und der Aktivierung wird eine Event-Nachricht über die erfolgreiche oder abgelehnte Fernprogrammierung an das Patientengerät übermittelt. Das Patientengerät leitet in Schritt 83 die Event-Nachricht weiter an das Servicecenter. In Schritt 85 wird die Event-Nachricht über die Fernprogrammierung verarbeitet. Eine Information hinsichtlich der Event-Nachricht geht an da Programmiergerät, das in Schritt 87 die Fernprogrammierung als abgeschlossen markiert. Die Weiterleitung der Information über die Event-Nachricht für Schritt 87 erfolgt beispielsweise bei der nächsten sich bietenden Gelegenheit und kann per LAN, WLAN oder GSM erfolgen. Eine weitere Information wird im Servicecenter selbst aktiviert, das eine Information in Schritt 89 an den Arzt über das HMSC veranlasst.

Ein alternativer Ablauf für eine asynchrone Fernprogrammierung ist in Fig. 7 schematisch dargestellt. Hierbei erfolgt die Fernprogrammierung ohne primäre Einschaltung eines Servicecenters bei der Übermittlung des Programmauftrags an das Patientengerät. Der in Fig. 7 dargestellte Ablauf entspricht weitgehend dem in Fig. 6 dargestellten Ablauf. Statt der Übermittlung an das Servicecenter erfolgt gemäß der Fig. 7 eine Übertragung per Mobilfunk über eine entsprechende Telefongesellschaft in Schritt 77' an das Patientengerät, wobei der Endanschluss an das Patientengerät auch über eine landgestützte Telefonleitung erfolgen kann. Der sonstige Ablauf des Programmiervorgangs entspricht dem, der anhand von Fig. 6 beschrieben wurde.

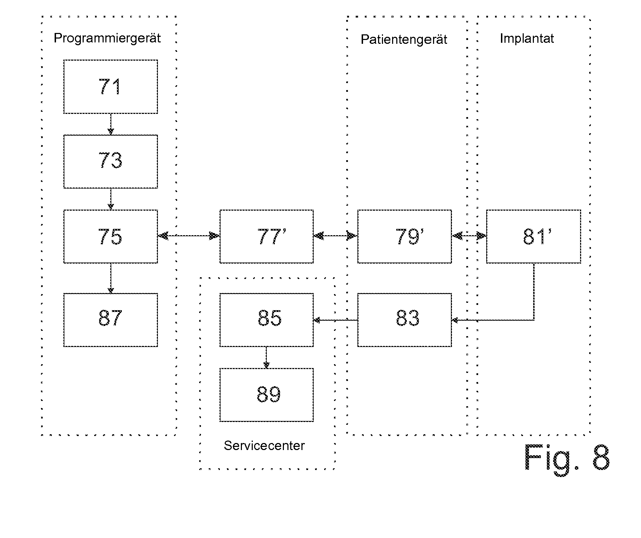

Fig. 8 zeigt schematisch einen Ablauf einer synchronen Fernprogrammierung mit einer Kopplung zwischen Programmiergerät und Patientengerät ohne Einschaltung eines Servicecenters zur Übermittlung von Daten an das Patientengerät. Im Grundsatz entspricht wiederum der in Fig. 8 dargestellte Ablauf weitgehend dem asynchronen Ablauf, wie er in Fig. 7 dargestellt ist. Wiederum leitet eine Telefongesellschaft oder ein ähnlicher Provider zur Übermittlung von Daten in Schritt 77' den Programmierauftrag vom Programmiergerät an das Patientengerät. In Schritt 79' wird allerdings nach Annehmen des Programms das Programm direkt an das Implantat weitergeleitet, was natürlich voraussetzt, dass das Implantat innerhalb der Reichweite des Patientengeräts ist. Eine Bestätigung über die Weiterleitung an das Implantat wird direkt, auf beispielsweise dem gleichen Wege, auf dem auch der Programmierauftrag an das Patientengerät gelangt ist, eine Bestätigung zurück an das Programmiergerät gesandt. Entsprechend kann auch auf dem entsprechenden Weg eine Bestätigung an das Programmiergerät gesandt werden, dass der Programmierauftrag für gültig befunden wurde und das Programm aktiviert wurde beziehungsweise dass die Programmaktivierung verweigert wurde (Schritt 81'). Wie schon bei den oben beschriebenen Abläufen geht eine Event-Nachricht über eine erfolgreiche Fernprogrammierung oder auch über eine verweigerte Fernprogrammierung an das Patientengerät und das Servicecenter, wodurch eine Information des Arztes veranlasst wird (Schritte 83, 85, 89). Da der Arzt in Folge der Synchronizität der Fernprogrammierung am Programmiergerät selbst eine Information darüber erhält, ob die Programmierung erfolgreich abgeschlossen wurde, kann der Arzt in Schritt 87' auch selbst die Fernprogrammierung abschließen.

Die Erstellung des neuen Programms einschließlich der Prüfung aller möglichen Konflikte erfolgt in für einen Arzt in gewohnter Weise am Programmiergerät wie bisher. Restriktionen bei der Einstellung der Parameter (z.B. Begrenzung des Einstellbereichs), die sich aus eventuellen Sicherheitsbedenken hinsichtlich der Tele-Übertragung ergeben, werden im Programmiergerät überprüft. Ein valider Parametersatz für die Tele-Programmierung kann alternativ zur Übertragung per Nahbereichstelemetrie an eine Relais-Station zur Weiterverteilung an das Ziel-Implantat übergeben werden. Das Programmiergerät überträgt das Programm dann beispielsweise entweder direkt an das Patientengerät oder über eine Web-Plattform, wozu es mit einem geeigneten Netzwerkinterface ausgerüstet ist (z.B. WLAN, LAN, GSM, UMTS, Bluetooth). Die Übertragung des Programms kann asynchron oder synchron erfolgen. Bei der synchronen Übertragung besteht während der Programmübertragung vom Arzt/Programmiergerät eine Verbindung zwischen dem Patienten und seinem Patientengerät. Die Programmübertragung und ggf. eine Rückmeldung erfolgen sofort. Bei der asynchronen Übertragung wird das geänderte Programm zunächst im Patientengerät abgelegt und erst in das Implantat transferiert, wenn das Implantat das nächste Mal in den Kommunikationsbereich des Patientengerätes gelangt. Es ist keine zeitliche Abstimmung zwischen Arzt und Patient erforderlich, dafür ist zunächst nicht vorbestimmt, wann das Programm im Implantat aktiv wird.

Die Fernprogrammierung setzt bevorzugt voraus, dass das aktuelle Programm des Implantats bekannt ist, d.h. das Programmiergerät kann dem Arzt das letzte bekannte Programm anzeigen, damit dieser seine Therapieentscheidung auf dieser Basis und korrekt treffen kann. Die Fernübertragung des Programms beinhaltet dabei außerdem eine Methode, durch die gewährleistet ist, dass das neue Programm im Implantat nur akzeptiert wird, wenn das aktuelle Implantatprogramm dem vom Programmiergerät vermuteten Programm entspricht.

## Patentansprüche

1. Anordnung für die Fernprogrammierung eines programmierbaren persönlichen Gerätes, insbesondere eines implantierbaren medizinischen Gerätes wie einem Herzschrittmacher, Defibrillator oder dergleichen, mit den Komponenten:
- programmierbares persönliches Gerät (10, 10')
- auf das persönliche Gerät abgestimmtes Programmiergerät (20) und
- auf das persönliche Gerät abgestimmtes Patientengerät (30)
von denen das persönliche Gerät (10)
eine erste Schnittstelle (11) zum wenigstens mittelbaren Verbinden des persönlichen Gerätes mit dem Patientengerät und
eine programmierbare Steuerung (13) für Funktionen des persönlichen Gerätes anhand von Steuerparametern aufweist, die mindestens dazu ausgebildet ist, einen Programmierauftrag über die erste Schnittstelle entgegenzunehmen
von denen das Programmiergerät (20)
eine erste Schnittstelle (21) zum wenigstens mittelbaren Verbinden des Programmiergerätes mit dem Patientengerät,
eine Programmiereinheit (23) für das persönliche Gerät, welche ausgebildet ist, Eingaben zum Programmieren, insbesondere zum Ändern, von Steuerparametern des persönlichen Gerätes entgegenzunehmen und diese Eingaben auf Plausibilität und Kompatibilität mit dem persönlichen Gerät hin zu überprüfen und nur einen mit diesem Gerät kompatiblen und plausiblen Programmierauftrag zuzulassen, und
eine Eingabeeinheit (25) für die Entgegennahme von Eingaben eines Benutzers aufweist, die mit der Programmiereinheit verbunden und mit dieser zusammen ausgebildet ist, Programmiereingaben zu machen, diese zu einem Programmierauftrag (51) zusammenzustellen und den Programmierauftrag über die erste Schnittstelle des Programmiergerätes an das Patientengerät zu übertragen, und
von denen das Patientengerät (30)
eine erste Schnittstelle (31) zum wenigstens mittelbaren Verbinden des Patientengerätes mit dem persönlichen Gerät und
eine zweite Schnittstelle (33) zum wenigstens mittelbaren Verbinden des Patientengerätes mit dem Programmiergerät aufweist,
wobei das Patientengerät (30) ausgebildet ist,
einen von dem Programmiergerät über die erste Schnittstelle empfangenen Programmierauftrag über die zweite Schnittstelle zu dem persönlichen Gerät zu übertragen.

2. Anordnung nach Anspruch 1, **dadurch gekennzeichnet, dass** das persönliche Gerät ein aktives medizinisches Implantat ist, insbesondere ein implantierbarer Herzschrittmacher oder Defibrillator-Kardioverter.

3. Anordnung nach Anspruch 2, **dadurch gekennzeichnet, dass** die erste Schnittstelle des Patientengerätes und die erste Schnittstelle des persönlichen Gerätes für eine drahtlose Kommunikation mit einer Reichweite im Bereich bis zu 5 m ausgebildet sind, insbesondere gemäß einer Medical Implant Communications Service-Spezifikation.

4. Anordnung nach einem der vorstehenden Ansprüche, **gekennzeichnet durch** ein Servicecenter (40) mit einer ersten Schnittstelle (41) zum Verbinden des Servicecenters mit dem Programmiergerät und einem Datenspeicher (43) für zumindest einem persönlichen Gerät zugehörige Daten,
wobei die erste Schnittstelle des Programmiergerätes für eine Verbindung mit der ersten Schnittstelle des Patientengerätes ausgebildet ist oder das Programmiergerät eine zweite Schnittstelle (29) zum Verbinden des Programmiergerätes mit dem Servicecenter aufweist,
wobei die Programmiereinheit und die Eingabeeinheit zusammen ausgebildet sind, von dem Servicecenter zu dem persönlichen Gerät gehörige Daten anzufordern.

5. Anordnung nach Anspruch 4, **dadurch gekennzeichnet, dass** das Servicecenter eine zweite Schnittstelle (45) zum wenigstens mittelbaren Verbinden des Servicecenters mit dem Patientengerät aufweist,
wobei das Programmiergerät ausgebildet ist, den Programmierauftrag an das Servicecenter zu übertragen und das Servicecenter ausgebildet ist, den empfangenen Programmierauftrag an das Patientengerät zu übertragen und / oder
- eine Dateneinheit (47) aufweist, die ausgebildet ist, Daten auf Basis des Programmierauftrages zu den im Datenspeicher gespeicherten dem persönlichen Gerät zugehörigen Daten hinzuzufügen und / oder
- eine Verifikationseinheit (49) aufweist, die ausgebildet ist, vor einer Übertragung von Daten an das Programmiergerät und/oder vor einer Übertragung des Programmierauftrages eine Verifikation des Programmiergerätes durchzuführen,

6. Anordnung nach einem der vorstehenden Ansprüche, **dadurch gekennzeichnet, dass** das Programmiergerät ausgebildet ist, dem Programmierauftrag eine Identifikation (53) eines vorherigen Programms beizugeben, insbesondere eine eindeutige Programmnummer oder ein aus dem vorherigen Programm abgeleitetes Kennzeichen,
wobei das persönliche Gerät eine Abgleichseinheit (15) aufweist, die ausgebildet ist, die dem Programmierauftrag beigegebene Identifikation mit dem aktuellen Programm der Steuerung abzugleichen und die Steuerung ausgebildet ist, im Fall eines negativen Abgleichsergebnisses den Programmierauftrag zurückzuweisen.

7. Anordnung nach einem der vorstehenden Ansprüche, **dadurch gekennzeichnet, dass** die Steuerung ausgebildet ist, im Fall einer erfolgten Ausführung des Programmierauftrags und/oder im Fall einer verweigerten Ausführung des Programmierauftrags eine Meldung an das Patientengerät und/oder das Programmiergerät zu übertragen.

8. Anordnung nach einem der vorstehenden Ansprüche, **dadurch gekennzeichnet, dass** das Patientengerät einen Programmspeicher (35) zum Zwischenspeichern des Programmierauftrages aufweist, wobei das Patientengerät ausgebildet ist, bei Herstellen einer Verbindung mit dem persönlichen Gerät den im Programmspeicher gespeicherten Programmierauftrag an das persönliche Gerät zu übermitteln.

9. Anordnung nach einem der vorstehenden Ansprüche, **dadurch gekennzeichnet, dass** das Programmiergerät eine dritte Schnittstelle (27) zum Verbinden mit dem persönlichen Gerät aufweist, insbesondere für eine drahtlose Kommunikation, vorzugsweise gemäß einer Medical Implant Communications Service-Spezifikation.

10. Anordnung nach einem der vorstehenden Ansprüche, **dadurch gekennzeichnet, dass** das Programmiergerät ausgebildet ist, dem Programmierauftrag eine Authentifizierung (55) beizugeben,
wobei das Patientengerät (30) und/oder das persönliche Gerät (10,10') eine Authentifizierungseinheit (37) aufweist, die dazu ausgebildet ist, den Programmierauftrag nur bei Vorliegen einer gültigen Authentifizierung an das persönliche Gerät übertragen zu lassen.

11. Anordnung nach einem der vorstehenden Ansprüche, **dadurch gekennzeichnet, dass** Programmiereinheit und Eingabeeinheit zusammen ausgebildet sind, einen Übertragungsweg für einen Programmierauftrag festzulegen,
wobei die Programmiereinheit ausgebildet ist, die Überprüfung auf Plausibilität und Kompatibilität in Abhängigkeit von dem festgelegten Übertragungsweg durchzuführen.

12. Anordnung nach Anspruch 11, **dadurch gekennzeichnet, dass** die Programmiereineinheit ausgebildet ist, für einen Übertragungsweg über die Patienteneinheit nur einen Programmierauftrag mit Steuerparametern in einem Bereich zuzulassen, der enger ist als ein Bereich, der für einen direkten Übertragungsweg vom Programmiergerät an das persönliche Gerät vorgesehen ist.

13. Programmiergerät (20), insbesondere zur Verwendung in einer Anordnung nach einem der vorstehenden Ansprüche, mit den Komponenten:
- erste Schnittstelle zum wenigstens mittelbaren Verbinden des Programmiergerätes mit einem Patientengerät
- Programmiereinheit für ein persönliches Gerät und
- Eingabeeinheit für die Entgegennahme von Eingaben eines Benutzers,
von denen die Programmiereinheit
ausgebildet ist, Eingaben zum Programmieren, insbesondere zum Ändern, von Steuerparametern des persönliches Gerätes entgegenzunehmen und diese Eingaben auf Plausibilität und Kompatibilität mit dem persönlichen Gerät hin zu überprüfen und nur einen mit diesem Gerät kompatiblen und plausiblen Programmierauftrag zuzulassen, und
von denen die Eingabeeinheit
mit der Programmiereinheit verbunden und mit dieser zusammen ausgebildet ist, Programmiereingaben zu machen, diese zu einem Programmierauftrag zusammenzustellen und den Programmierauftrag über die erste Schnittstelle des Programmiergerätes an das Patientengerät zu übertragen.

14. Patientengerät (30), insbesondere zur Verwendung in einer Anordnung nach einem der Ansprüche 1 bis 12, mit den Komponenten:
- erste Schnittstelle zum wenigstens mittelbaren Verbinden des Patientengerätes mit einem persönlichen Gerät und
- zweite Schnittstelle zum wenigstens mittelbaren Verbinden des Patientengerätes mit einem Programmiergerät,
wobei das Patientengerät ausgebildet ist,
einen von dem Programmiergerät über die erste Schnittstelle empfangenen Programmierauftrag über die zweite Schnittstelle zu dem persönlichen Gerät zu übertragen.

15. Servicecenter (40), insbesondere zur Verwendung in einer Anordnung nach einem der Ansprüche 1 bis 12, mit den Komponenten,
- erste Schnittstelle (41) zum Verbinden des Servicecenters mit einem Programmiergerät
- Datenspeicher (43) für zumindest einem persönlichen Gerät zugehörige Daten,
- zweite Schnittstelle (45) zum wenigstens mittelbaren Verbinden des Servicecenters mit einem Patientengerät,
wobei der Datenspeicher ausgebildet ist, auf Anforderung eine Übertragung von zu dem persönlichen Gerät gehörigen Daten über die erste Schnittstelle zu veranlassen, und
wobei das Servicecenter ausgebildet ist, einem von einem Programmiergerät empfangenen Programmierauftrag an das Patientengerät zu übertragen.

16. Verfahren für die Fernprogrammierung eines programmierbaren persönlichen Gerätes, insbesondere eines implantierbaren medizinischen Gerätes wie einem Herzschrittmacher, Defibrillator oder dergleichen, mittels einer Anordnung nach einem der Ansprüche 1 bis 12, mit den Verfahrensschritten:
- Auswählen (61) eines persönlichen Gerätes,
- Zusammenstellen (63) eines Programmierauftrags durch Entgegennehmen von Eingaben eines Benutzers und Überprüfen (63) der Eingaben auf Plausibilität und Kompatibilität mit dem persönlichen Gerät in einem Programmiergerät,
- Übertragen (65) des Programmierauftrags an ein auf das persönliche Gerät abgestimmtes Patientengerät über eine wenigstens mittelbare Verbindung,
- Empfangen (67) des Programmierauftrags durch das Patientengerät und Übertragen des Programmierauftrags an das persönliche Gerät,
- Entgegennehmen (69) des Programmierauftrags durch eine programmierbare Steuerung des persönlichen Gerätes.
